# EUROPÄISCHE PATENTSCHRIFT

(11) **EP 3 770 140 B1**
(45) Veröffentlichungstag und Bekanntmachung des Hinweises auf die Patenterteilung: **14.12.2022**
(21) Anmeldenummer: 19188698.5
(22) Anmeldetag: 26.07.2019
(51) Int. Cl.: C07C 29/151, C07C 31/04, B01J 8/00, B01J 8/04, B01J 12/00, C01B 3/36, C01B 3/38

(54) **VERFAHREN UND ANLAGE ZUR SYNTHESE VON METHANOL**
METHOD AND SYSTEM FOR SYNTHESISING METHANOL
PROCÉDÉ ET INSTALLATION DE SYNTHÈSE DE MÉTHANOL

(43) Veröffentlichungstag der Anmeldung: 27.01.2021
(73) Patentinhaber: Balthasar, Wolff, 40833 Ratingen (DE); Koss, Ulrich, 61348 Bad Homburg vor der Höhe (DE); Wagner, Ulrich, 06406 Bernburg (DE); Rappold, Dorit, 60437 Frankfurt (DE); Lauer, Wolfgang, 34613 Schwalmstadt (DE)
(72) Erfinder: Balthasar, Wolff, 40833 Ratingen (DE); Koss, Ulrich, 61348 Bad Homburg vor der Höhe (DE); Wagner, Ulrich, 06406 Bernburg (DE); Rappold, Dorit, 60437 Frankfurt (DE); Lauer, Wolfgang, 34613 Schwalmstadt (DE)
(74) Vertreter: Patentanwälte Bauer Vorberg Kayser

(56) Entgegenhaltungen:
- EP-A1- 2 116 295

## Beschreibung

Die Erfindung betrifft ein Verfahren zur Synthese von Methanol gemäß dem Oberbegriff von Patentanspruch 1, ein Verfahren zum Umbau einer Anlage zur Synthese von Methanol gemäß dem Oberbegriff von Patentanspruch 15 sowie eine Anlage zur Synthese von Methanol gemäß dem Oberbegriff von Patentanspruch 16.

Bei der Methanolproduktion aus Erdgas wird regelmäßig in einem ersten Schritt aus dem Erdgas ein Synthesegas gewonnen, welches Synthesegas dann in einem anschließenden Schritt zu Methanol synthetisiert wird. Ein herkömmlicher Ansatz zum Gewinnen des Synthesegases besteht darin, das Erdgas in einem Dampfreformer einer Dampfreformierung unter Zusatz von Wasserdampf zu unterziehen. Die darauf folgende Methanolsynthese aus dem Synthesegas findet dann in einem Methanolreaktor statt. Aus dem Stand der Technik ist es ebenso bekannt, zur Erhöhung des Durchsatzes einer Anlage zur Synthese von Methanol eine Vielzahl von Dampfreformern vorzusehen, wobei jeder Dampfreformer jeweils einen eigenen Methanolreaktor mit dem in ihm erzeugten Frischgasstrom speist. Man spricht in so einem Fall dann von einer Vielzahl von Strängen für die Methanolsynthese, wobei jeder Strang einen Dampfreformer und den zugehörigen Methanolreaktor umfasst.

Nachteilig an diesem Ansatz ist, dass das bei der Dampfreformierung erzeugte Synthesegas eine Stöchiometrie aufweist, welche für die Methanolsynthese nicht ideal ist. Speziell weist das bei der Dampfreformierung erzeugte Synthesegas einen Überschuss an Wasserstoff auf, sodass also das Mengenverhältnis zwischen Wasserstoff und den Kohlenstoffoxiden überhöht ist. Das Synthesegas wird daher als überstöchiometrisch bezeichnet. Dieser Überschuss führt dazu, dass eine vergleichsweise große Menge an unreagiertem Wasserstoff aus dem Methanolreaktor austritt. Um zu verhindern, dass deswegen sehr viel unreagiertes Gas im Synthesekreislauf gefahren und auch aufwändig komprimiert werden muss, wird ein Teil des unreagierten Wasserstoffs gemeinsam mit nicht in Synthesegas umgewandeltem Methan als Purgegas ausgekreist. Das Purgegas wird dann regelmäßig zur Befeuerung des Dampfreformers verwendet. Im Ergebnis ist wegen der Verbrennung von erzeugtem, aber nicht genutzten Wasserstoff und der erhöhten Menge an zirkulierendem Gas der Energieverbrauch bei der Methanolsynthese und damit auch die Abgabe von Kohlenstoffdioxid als Abgas bei diesem Ansatz deutlich erhöht.

Die EP 2 116 295 A1, von welcher die vorliegende Erfindung ausgeht, versucht dadurch eine für die Methanolsynthese geeignetere Stöchiometrie zu erreichen, dass ein Synthesegas nicht nur in einem Dampfreformer aus Erdgas gewonnen wird, sondern zusätzlich auch das Gewinnen von weiterem Synthesegas durch nicht katalytische partielle Oxidation in einem POX-Reaktor vorgesehen ist. Das durch nicht katalytische partielle Oxidation aus Erdgas gewonnene Synthesegas ist dabei - im Gegensatz zu dem Synthesegas aus der Dampfreformierung - unterstöchiometrisch und weist daher ein Wasserstoffdefizit auf. Weiter kann hier nun das Synthesegas aus der Dampfreformierung derart mit dem Synthesegas aus dem POX-Reaktor vermischt werden, dass das resultierende Synthesegas die gewünschte Stöchiometrie für die Methanolsynthese erreicht. Entsprechend werden hier die beiden Synthesegasströme in einem gemeinsamen Methanolreaktor für die Methanolsynthese zusammengeführt.

Grundsätzlich ist dieser Ansatz zwar dafür tauglich, in dem gemeinsamen Methanolreaktor eine verbesserte Stöchiometriezahl zu erreichen. Jedoch ist der Ansatz wenig hilfreich dabei, die Wirtschaftlichkeit bereits vorhandener Anlagen für die Methanolsynthese auf Basis von Dampfreformierung - insbesondere wenn sie mehrere Stränge aufweisen - verbessern zu können. Zu weitreichend sind dann nämlich die erforderlichen Umbauten in der Gesamtanlage.

Ausgehend von diesem Stand der Technik besteht die Aufgabe der Erfindung folglich darin, einen Ansatz für die Methanolsynthese bereitzustellen, welcher neben der Verbesserung der Wirtschaftlichkeit durch deutliche Reduzierung des spezifischen Erdgaseinsatzes für die Methanolsynthese eine gleichzeitige signifikante Reduzierung der Emissionen von Kohlenstoffdioxid bei bereits bestehenden und insbesondere mehrzügigen Anlagen auf Basis von Dampfreformierung ermöglicht.

Bezogen auf ein Verfahren zur Synthese von Methanol mit den Merkmalen des Oberbegriffs von Anspruch 1 wird diese Aufgabe durch die Merkmale des kennzeichnenden Teils von Anspruch 1 gelöst. Bezogen auf ein Verfahren zum Umbau einer Anlage zur Synthese von Methanol mit den Merkmalen des Oberbegriffs von Anspruch 15 wird diese Aufgabe durch die Merkmale des kennzeichnenden Teils von Anspruch 15 und bezogen auf eine Anlage zur Synthese von Methanol mit den Merkmalen des Oberbegriffs von Anspruch 16 wird diese Aufgabe durch die Merkmale des kennzeichnenden Teils von Anspruch 16 gelöst.

Der Erfindung liegt die Erkenntnis zugrunde, dass es auch bei der separaten Herstellung von überstöchiometrischem Gas durch Dampfreformierung einerseits und der Herstellung von unterstöchiometrischem Gas durch partielle Oxidation andererseits dennoch vorteilhaft ist, die entsprechenden Frischgasströme zunächst ebenfalls separaten Methanolreaktoren zuzuführen. Die Frischgasströme verbleiben also in ihrem jeweiligen Strang. Ein Ausgleich der Stöchiometrie kann dann dadurch erfolgen, dass das weiterhin einen Wasserstoffüberschuss aufweisende Restgas aus dem Methanolreaktor der Dampfreformierung teilweise dem Methanolreaktor der partiellen Oxidation zugeführt wird, und zwar direkt oder indirekt. Erst das Restgas wird also von einem Strang zum anderen abgezweigt. Auf diese Weise ist es zur Verbesserung der Wirtschaftlichkeit lediglich erforderlich, einen Reaktor für die partielle Oxidation sowie einen dazugehörigen Methanolreaktor zusätzlich oder anstelle eines vorhandenen Dampfreformers und des dazugehörigen Methanolreaktors vorzusehen. Alle weiteren Stränge aus Dampfreformer und Methanolreaktor können im Wesentlichen unverändert belassen werden.

Dabei kann die Zuführung des Wasserstoffs zu dem unterstöchiometrischen Methanolreaktor der partiellen Oxidation auf verschiedene Art erfolgen. Die Unteransprüche 3, 5 und 6 stellen sowohl eine direkte als auch indirekte Varianten vor.

Wie der Unteranspruch 8 feststellt, kann auch hier ein Teil des unreagierten Wasserstoffs für die Befeuerung der Dampfreformierung benutzt werden, wobei der Unteranspruch 9 vorteilhafte Anteilswerte beschreibt. Gemäß Unteranspruch 11 kann für eine solche Befeuerung auch Restgas aus dem Methanolreaktor der partiellen Oxidation verwendet werden.

Der Unteranspruch 12 beschreibt bevorzugte Ausgestaltungen, die eine besonders effektive Ausnutzung der Kompressoren vorsehen. Da der Energieverbrauch bei der Druckerhöhung einen besonders relevanten Kostenfaktor darstellt, machen sich Einsparungen in diesem Bereich deutlich bei der Beurteilung der Wirtschaftlichkeit bemerkbar.

Eine Verringerung des zu führenden und im Kreislauf zu fahrenden Prozessgases lässt sich auch dadurch erreichen, dass die partielle Oxidation mit einem Sauerstoffstrom durchgeführt wird, welcher aus einer Luftzerlegung gewonnen wurde. Dies beschreibt der Unteranspruch 13.

Schließlich betrifft der Unteranspruch 14 ein Szenario mit mehr als zwei Zügen für die Methanolsynthese.

Bevorzugte Ausgestaltungen, Merkmale und Vorteile des erfindungsgemäßen Verfahrens zur Synthese von Methanol entsprechen bevorzugten Ausgestaltungen, Merkmalen und Vorteilen der erfindungsgemäßen Anlage zur Synthese von Methanol sowie des erfindungsgemäßen Verfahrens zum Umbau einer Anlage zur Synthese von Methanol und umgekehrt.

Weitere Einzelheiten, Merkmale, Ziele und Vorteile der vorliegenden Erfindung werden nachfolgend anhand einer lediglich Ausführungsbeispiele wiedergebenden Zeichnung erläutert. In der Zeichnung zeigt
- Fig. 1: eine schematische Darstellung eines ersten Ausführungsbeispiels der vorschlagsgemäßen Anlage zur Synthese von Methanol zur Ausführung des vorschlagsgemäßen Verfahrens zur Synthese von Methanol,
- Fig. 2: eine schematische Darstellung eines zweiten Ausführungsbeispiels der vorschlagsgemäßen Anlage zur Synthese von Methanol zur Ausführung des vorschlagsgemäßen Verfahrens zur Synthese von Methanol,
- Fig. 3: eine schematische Darstellung eines dritten Ausführungsbeispiels der vorschlagsgemäßen Anlage zur Synthese von Methanol zur Ausführung des vorschlagsgemäßen Verfahrens zur Synthese von Methanol,
- Fig. 4: eine schematische Darstellung eines adiabaten Quenchreaktors zur Methanolsynthese als Bestandteil der jeweiligen vorschlagsgemäßen Anlage der Fig. 1 bis 3 sowie 6,
- Fig.5: eine schematische Darstellung eines isothermen Reaktors zur Methanolsynthese als Bestandteil der jeweiligen vorschlagsgemäßen Anlage der Fig. 1 bis 3 sowie 6 und
- Fig. 6: eine schematische Darstellung eines vierten Ausführungsbeispiels der vorschlagsgemäßen Anlage zur Synthese von Methanol zur Ausführung des vorschlagsgemäßen Verfahrens zur Synthese von Methanol.

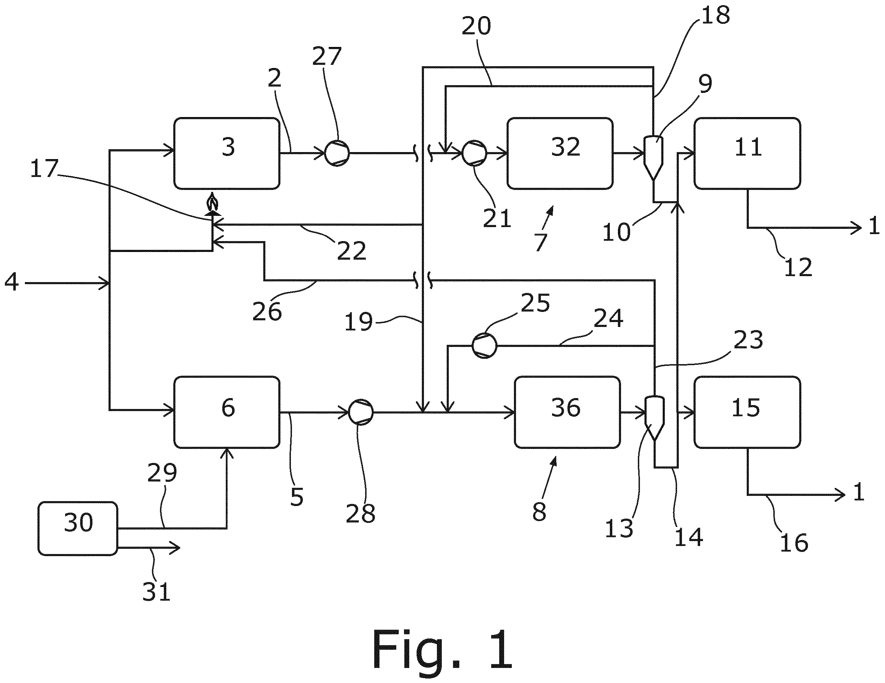

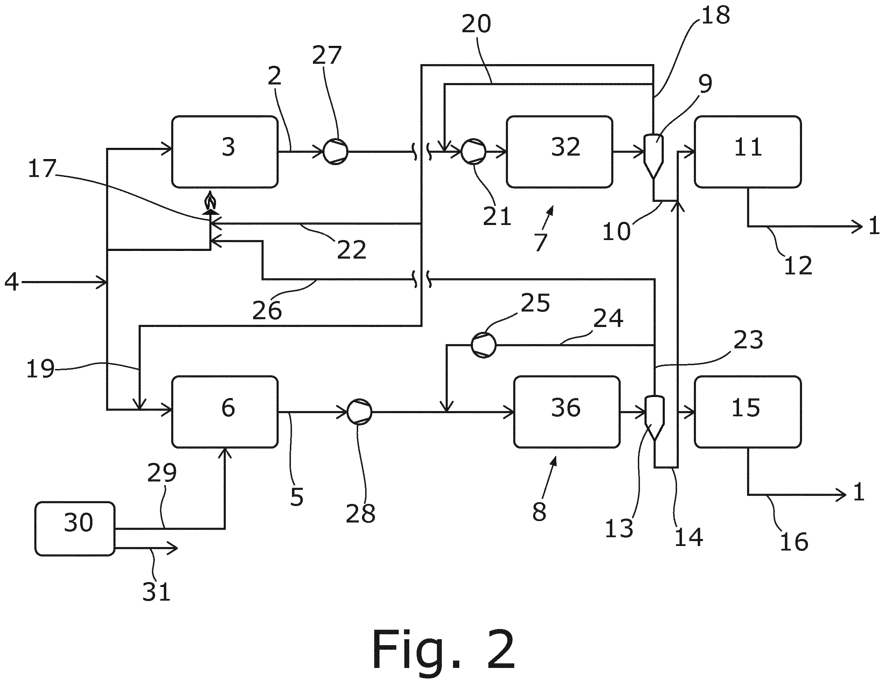

Das vorschlagsgemäße Verfahren zur Synthese von Methanol 1 kann durch eine der vorschlagsgemäßen Anlagen zur Synthese von Methanol 1 gemäß den Ausführungsbeispielen der Fig. 1 bis 3 und 6 durchgeführt werden. Das vorschlagsgemäße Verfahren zur Synthese von Methanol 1 wird nachstehend zunächst anhand der vorschlagsgemäßen Anlage zur Synthese von Methanol 1 aus der Fig. 1 erläutert. Grundsätzlich basieren die Ausführungsbeispiele der Fig. 2, 3 und 6 auf dem Ausführungsbeispiel der Fig. 1, wobei auf bestehende Unterschiede hingewiesen wird.

Beim vorschlagsgemäßen Verfahren zur Synthese von Methanol 1 wird ein erster Frischgasstrom 2 mit Wasserstoff und Kohlenstoffoxiden in einem Dampfreformer 3 durch Dampfreformierung aus einem kohlenstoffhaltigen Energieträgerstrom 4 gewonnen. Bei dem kohlenstoffhaltigen Energieträgerstrom 4 handelt es sich hier und vorzugsweise um einen Erdgasstrom umfassend Methan, Ethan und Propan.

Beim vorschlagsgemäßen Verfahren zur Synthese von Methanol 1 wird weiter ein zweiter Frischgasstrom 5 mit Wasserstoff und Kohlenstoffoxiden in einem POX-Reaktor 6 durch partielle Oxidation aus dem kohlenstoffhaltigen Energieträgerstrom 4 gewonnen. Grundsätzlich kann es sich bei dieser partiellen Oxidation sowohl um katalytische partielle Oxidation als auch um thermische partielle Oxidation. Diese thermische partielle Oxidation kann, in Abgrenzung zur katalytischen partiellen Oxidation, auch als nicht katalytische partielle Oxidation bezeichnet werden. Im vorliegenden Fall und bevorzugt dient der POX-Reaktor 6 der thermischen partiellen Oxidation.

Ebenso wird beim vorschlagsgemäßen Verfahren zur Synthese von Methanol 1 der erste Frischgasstrom 2 einer ersten Methanolreaktoranordnung 7 zur Synthese von Methanol 1 zugeführt. Das vorschlagsgemäße Verfahren zur Synthese von Methanol 1 ist dadurch gekennzeichnet, dass der zweite Frischgasstrom 5 einer zweiten Methanolreaktoranordnung 8 zur Synthese von Methanol 1 zugeführt wird.

Wie in den Fig. 1 bis 3 und 6 gezeigt und bevorzugt wird aus einer ersten Kondensationsvorrichtung 9 der ersten Methanolreaktoranordnung 7 ein erster Rohmethanolstrom 10 erhalten, welcher einer ersten Destillationsanordnung 11 zum Gewinnen eines ersten Methanolproduktstroms 12 umfassend das Methanol 1 zugeführt wird. Entsprechend wird vorzugsweise aus einer zweiten Kondensationsvorrichtung 13 der zweiten Methanolreaktoranordnung 8 ein zweiter Rohmethanolstrom 14 erhalten, welcher einer zweiten Destillationsanordnung 15 zum Gewinnen eines zweiten Methanolproduktstroms 16 umfassend das Methanol 1 zugeführt wird.

Bevorzugt ist, dass der Dampfreformer 3 durch eine befeuerte Heizvorrichtung 17 erhitzt wird. Diese befeuerte Heizvorrichtung 17 ist von der Anlage zur Synthese von Methanol 1 umfasst. Vorzugsweise wird die befeuerte Heizvorrichtung 17 durch den kohlenstoffhaltigen Energieträgerstrom 4 gespeist wird. Durch die befeuerte Heizvorrichtung 17 kann die für die endotherme Reformierungsreaktion erforderliche Hitze bereitgestellt werden.

Neben dem kohlenstoffhaltigen Energieträgerstrom 4 können alternativ oder zusätzlich auch andere Quellen zum Speisen der befeuerten Heizvorrichtung 17 vorgesehen sein. Dies wird untenstehend noch genauer ausgeführt.

Wie bereits oben festgestellt wurde, weist der erste Frischgasstrom 2 für die Methanolsynthese einen Überschuss an Wasserstoff auf, sodass auch nach Durchlauf der ersten Methanolreaktoranordnung 7 im Restgas ein Überschuss an Wasserstoff verbleibt. Für den zweiten Frischgasstrom 5 und das Restgas der zweiten Methanolreaktoranordnung 8 stellt sich dies genau umgekehrt dar.

Entsprechend ist das vorschlagsgemäße Verfahren weiter dadurch gekennzeichnet, dass ein Restgasstrom 18 mit unreagiertem Wasserstoff und unreagierten Kohlenstoffoxiden aus der ersten Methanolreaktoranordnung 7 gewonnen wird und dass zumindest ein Teil des unreagierten Wasserstoffs der zweiten Methanolreaktoranordnung 8 zur Synthese von Methanol zugeführt wird. Dieser Sachverhalt ist auch in den Fig. 1 bis 3 und 6 dargestellt. Es kann auch sein, dass im Wesentlichen der vollständige unreagierte Wasserstoff des Restgasstroms 18 der zweiten Methanolreaktoranordnung 8 zur Synthese von Methanol zugeführt wird.

Der Restgasstrom 18 kann insbesondere aus der ersten Kondensationsvorrichtung 9 der ersten Methanolreaktoranordnung 7 gewonnen werden. Die Zufuhr des obigen Teils des unreagierten Wasserstoffs kann dabei auf verschiedene Arten und insbesondere sowohl direkt als auch indirekt erfolgen.

Grundsätzlich reicht es aus, wenn nur der unreagierte Wasserstoff des Restgasstroms 18 - also ohne die weiteren Bestandteile des Restgasstroms 18 - zumindest teilweise der zweiten Methanolreaktoranordnung 8 zugeführt wird. Bevorzugt ist, dass auch zumindest ein Teil der unreagierten Kohlenstoffoxide des Restgasstroms 18 der zweiten Methanolreaktoranordnung 8 zur Synthese von Methanol 1 zugeführt wird.

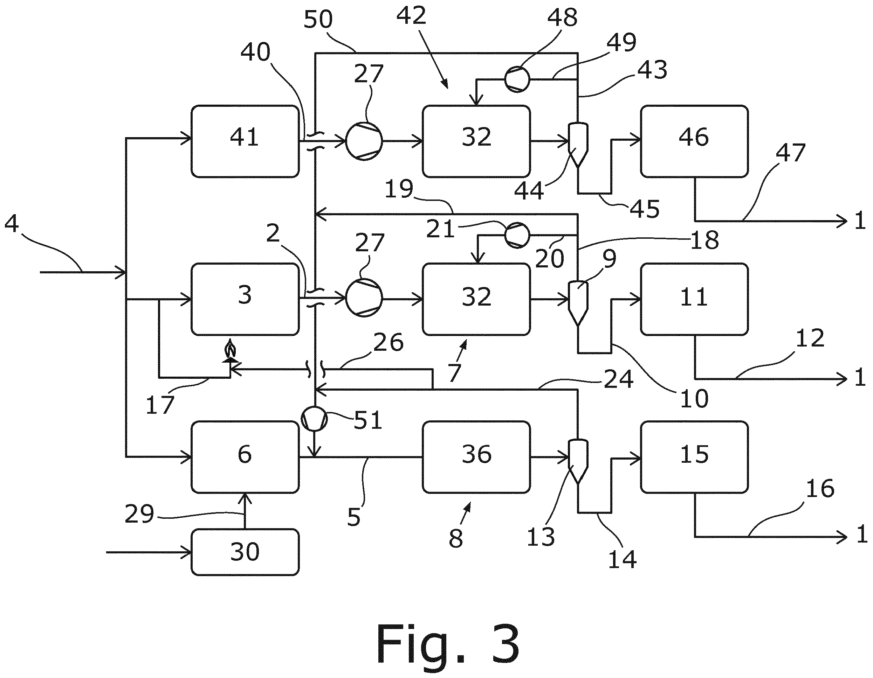

Eine erste bevorzugte Variante, welche den Ausführungsbeispielen der Fig. 1 und 3 entspricht, ist dadurch gekennzeichnet, dass ein erster Teilstrom 19 des Restgasstroms 18 der zweiten Methanolreaktoranordnung 8 zur Synthese von Methanol zugeführt wird. Es wird also hier der erste Teilstrom 19 dem zweiten Frischgasstrom 5 zugeführt, wobei dies wie hier dargestellt und bevorzugt prozesstechnisch nachgelagert zu einer noch genauer zu beschreibenden Druckerhöhung des zweiten Frischgasstroms 5 erfolgt. Hier wird also, wie oben bereits erwähnt, ein Teil des gesamten Restgasstroms 18 und damit auch ein Teil der unreagierten Kohlenstoffoxide des Restgasstroms 18 der zweiten Methanolreaktoranordnung 8 zugeführt. Denkbar ist, dass der erste Teilstrom 19 druckerhöht der zweiten Methanolreaktoranordnung 8 zugeführt, wobei diese Variante in dem Ausführungsbeispiel der Fig. 3 verwirklicht ist. Im Ausführungsbeispiel der Fig. 1 ist der Druck in der ersten Methanolreaktoranordnung 7 nahezu 20 bar höher als der Druck in der zweiten Methanolreaktoranordnung 8, sodass auch nach Durchlaufen der ersten Kondensationsvorrichtung der erste Teilstrom 19 einen hinreichend hohen Druck aufweist.

Soweit hier und nachfolgend von einem Teilstrom eines bestimmten Stroms die Rede ist, so kann es sich bei dem Teilstrom auch um den gesamten des bestimmten Stroms handeln. Voraussetzung ist, dass es keinen weiteren Teilstrom des bestimmten Stroms gibt. Entsprechend sieht eine bevorzugte Variante gemäß der jeweiligen Darstellung in der Fig. 1 und 3 vor, dass im Wesentlichen der vollständige erste Teilstrom 19 insbesondere mit im Wesentlichen gleichbleibender Zusammensetzung der zweiten Methanolreaktoranordnung 8 zur Synthese von Methanol 1 zugeführt wird. Die im Wesentlichen gleichbleibende Zusammensetzung des ersten Teilstroms 19 bedeutet, dass dieser erste Teilstrom 19 bzw. seine Bestandteile vor der Zuführung zu der zweiten Methanolreaktoranordnung 8 keine wesentliche Reaktion durchlaufen. Ein Beispiel für eine Änderung der Zusammensetzung bietet die nachfolgend beschriebene Variante.

Eine zweite bevorzugte Variante zur Zuführung zumindest eines Teils des unreagierten Wasserstoffs des Restgasstroms 18 sowie hier auch eines Teils der unreagierten Kohlenstoffoxide ist in dem zweiten Ausführungsbeispiel der Fig. 2 gezeigt. Diese Variante ist dadurch gekennzeichnet, dass der erste Teilstrom 19 des Restgasstroms 8 dem POX-Reaktor 6 zum Gewinnen des zweiten Frischgasstroms 5 zugeführt wird. Speziell kann diese Zuführung indirekt erfolgen, und zwar dadurch, dass der erste Teilstrom 19 dem kohlenstoffhaltigen Energieträgerstrom vor Zuführung zu dem POX-Reaktor 6 zugeführt wird. Eine Zuführung zu dem POX-Reaktor 6 ist für die Reaktion in dem POX-Reaktor 6 unschädlich, da Wasserstoff und Kohlenstoffoxide ohnehin die Edukte der partiellen Oxidation im POX-Reaktor 6 sind.

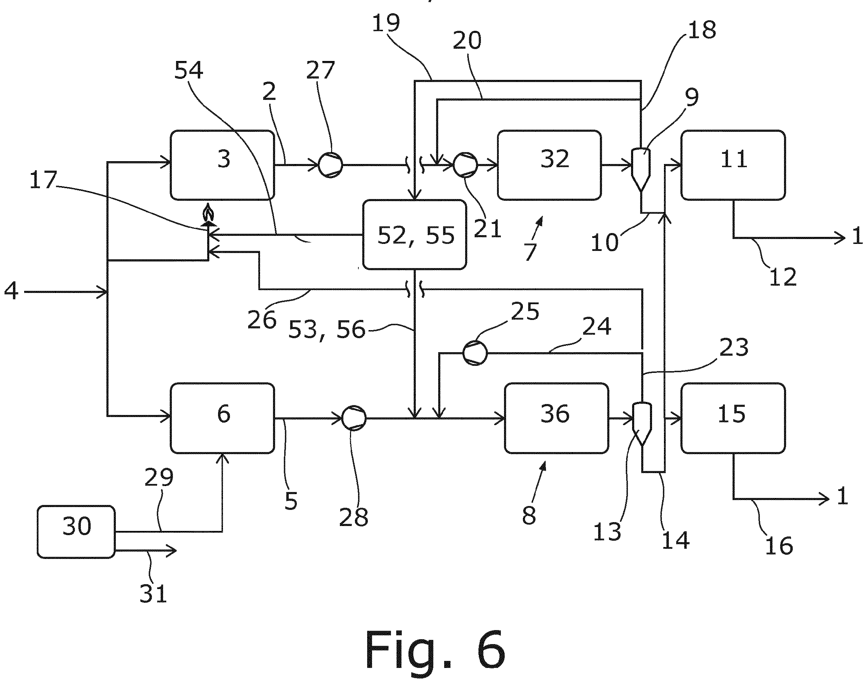

Eine dritte bevorzugte Variante zur Zuführung zumindest eines Teils des unreagierten Wasserstoffs des Restgasstroms 18 ist in dem vierten Ausführungsbeispiel der Fig. 6 gezeigt. Entsprechend dieser Variante ist bevorzugt vorgesehen, dass ein erster Teilstrom 19 des Restgasstroms 18 einer Wasserstoffrückgewinnungsanordnung 52 zum Gewinnen eines wasserstoffhaltigen Stroms 53 und eines Reststroms 54 zugeführt wird. Gemäß der in der Fig. 6 dargestellten Variante und wie bevorzugt wird der wasserstoffhaltige Strom 53 d er zweiten Methanolreaktoranordnung 8 zur Synthese von Methanol 1 zugeführt. Es ist aber auch möglich, dass der wasserstoffhaltige Strom 53 dem POX-Reaktor 6 zum Gewinnen des zweiten Frischgasstroms 5 zugeführt wird. Bezüglich des Reststroms 54 ist bevorzugt vorgesehen, dass er zum Speisen der befeuerten Heizvorrichtung 17 dieser zugeführt wird. Dies ist in der Fig. 6 auch dargestellt.

Grundsätzlich kann die Wasserstoffrückgewinnungsanordnung 52 auf beliebige Art und Weise den wasserstoffhaltigen Strom 53 gewinnen. Im Grunde kann der wasserstoffhaltige Strom 53 einen beliebigen Wasserstoffanteil aufweisen. Bevorzugt ist, dass der wasserstoffhaltige Strom 53 einen höheren molaren Anteil an Wasserstoff aufweist als der Restgasstrom 18. Es kann aber auch sein, dass der wasserstoffhaltige Strom 53 im Wesentlichen aus Wasserstoff besteht. Bei dem Reststrom 54 handelt es sich vorzugsweise um einen gegenüber dem Restgasstrom 18 molar an Methan, Kohlenstoffmonoxid und/oder Kohlenstoffdioxid angereicherten Strom.

Wie in dem Ausführungsbeispiel der Fig. 6 dargestellt, kann die Wasserstoffrückgewinnungsanordnung 52 eine Druckwechsel-Adsorptionsvorrichtung (PSA) 55 zum Gewinnen des wasserstoffhaltigen Stroms 53 umfassen. Wegen der dann erreichten hohen Reinheit handelt es sich bei dem wasserstoffhaltigen Strom um einen Wasserstoffstrom 56. Speziell wird der wasserstoffhaltige Strom 53 auf einer Hochdruckseite der Wasserstoffrückgewinnungsanordnung 52 gewonnen. Entsprechend wird der Reststrom 54 auf einer Niederdruckseite der Wasserstoffrückgewinnungsanordnung 52 gewonnen. Diese Ausgestaltung hat erstens den Vorteil, dass Methan im ersten Teilstrom 19 praktisch vollständig aus dem Kreislauf der Methanolsynthese entfernt wird. Zweitens kann der erreichte hohe Reinheitsgrad an Wasserstoff für die gewünschte Einstellung der Stöchiometrie besonders geeignet sein. Schließlich wird der wasserstoffhaltige Strom 53 mit einem vergleichsweise geringen Druckverlust gewonnen.

Die Wasserstoffrückgewinnungsanordnung 52 kann aber auch eine Membrananordnung oder eine sonstige Vorrichtung zum Gewinnen des wasserstoffhaltigen Stroms 53 umfassen.

Neben dem obigen ersten Teilstrom 19 des Restgasstroms 18 können auch weitere Teilströme des Restgasstroms 18 vorgesehen sein. Vorzugsweise wird ein zweiter Teilstrom 20 des Restgasstroms 18 zur ersten Methanolreaktoranordnung 7 zur Synthese von Methanol zurückgeführt. Diese Zurückführung kann gemäß der Darstellung in den Fig. 1, 2 und 6 dadurch erfolgen, dass der zweite Teilstrom 20 mit dem ersten Frischgasstrom 2 zusammengeführt wird. Bevorzugt erfolgt diese Zurückführung des zweiten Teilstroms 20 des Restgasstroms 18 druckerhöht. Wie ebenfalls in den Fig. 1, 2 und 6 dargestellt, kann diese Druckerhöhung durch einen von der Anlage zur Synthese von Methanol 1 umfassten Zirkulationskompressor 21 erfolgen, welcher sowohl den zweiten Teilstrom 20 des Restgasstroms 18 als auch den ersten Frischgasstrom 2 nach ihrer Zusammenführung druckerhöht.

Denkbar wäre aber auch, dass der Zirkulationskompressor 21 zur Druckerhöhung des zweiten Teilstroms 20 des Restgasstroms 18 vor der Zusammenführung mit dem ersten Frischgasstrom 2 vorgesehen ist. Eine solche Anordnung ist bei dem Ausführungsbeispiel der Fig. 3 gezeigt.

Eine Möglichkeit, das nicht in der Dampfreformierung umgesetzte Methan aus dem Kreislauf der Methanolsynthese zu entfernen besteht darin, einen Teil des Restgasstroms 18 zum Speisen der befeuerten Heizvorrichtung 17 zu verwenden. Daher ist es bevorzugt, dass ein dritter Teilstrom 22 des Restgasstroms 18 aus dem Restgasstrom 18 gewonnen wird. Dieser dritte Teilstrom 22 des Restgasstroms 18 kann dann der befeuerten Heizvorrichtung 17 zum Speisen der befeuerten Heizvorrichtung 17 zugeführt werden. Auf diese Weise findet auch eine Verwertung und Umwandlung in Kohlenstoffdioxid des Methans in dem dritten Teilstrom 22 statt. Das Entfernen aus dem Kreislauf desjenigen Methans, welches durch den ersten Teilstrom 19 der zweiten Methanolreaktoranordnung 8 zugeführt wurde, wird untenstehend noch beschrieben.

Grundsätzlich kann die Aufteilung des Restgasstroms 18 bezüglich der mengenmäßigen Anteile beliebig erfolgen. Jedoch ist bevorzugt, dass ein Massendurchsatz des dritten Teilstroms 22 niedriger ist als ein Massendurchsatz des ersten Teilstroms 19. Der Massendurchsatz kann dabei insbesondere durch die Größe Masse pro Zeiteinheit und insbesondere durch die Einheit Kilogramm pro Minute oder Tonnen pro Stunde gegeben sein. Speziell ist bevorzugt, dass der Massendurchsatz des dritten Teilstroms 22 zwischen 25 % und 70 % des Massendurchsatzes des ersten Teilstroms 19 beträgt. Genauer kann es sein, dass der Massendurchsatz des dritten Teilstroms 22 zwischen 40 % und 50 % des Massendurchsatzes des ersten Teilstroms 19 beträgt. Im Ausführungsbeispiel der Fig. 1 beträgt das Verhältnis des Massendurchsatzes des dritten Teilstroms 22 zu dem Massendurchsatz des ersten Teilstroms 19 30 zu 70. Es hat sich herausgestellt, dass ein solches Aufteilungsverhältnis sowohl eine geeignete Einstellung der Stöchiometrie für die Methanolsynthese bewirkt als auch einen effizienten Betrieb der befeuerten Heizvorrichtung 17 erlaubt.

Das oben beschriebene Rezirkulieren von unreagiertem Restgas für die erste Methanolreaktoranordnung 7 durch den zweiten Teilstrom 20 des Restgasstroms 18 kann auch für die zweite Methanolreaktoranordnung 8 vorgesehen sein. Entsprechend ist es gemäß den Darstellungen in den Fig. 1 bis 3 sowie 6 bevorzugt, dass ein weiterer Restgasstrom 23 mit unreagiertem Wasserstoff und unreagierten Kohlenstoffoxiden aus der zweiten Methanolreaktoranordnung 8 gewonnen wird und dass ein erster Teilstrom 24 des weiteren Restgasstroms 23 insbesondere druckerhöht zur zweiten Methanolreaktoranordnung 8 zur Synthese von Methanol 1 zurückgeführt wird. Im Ausführungsbeispiel der Fig. 3 entspricht der erste Teilstrom 24 dem vollständigen weiteren Restgasstrom 23. Vorzugsweise erfolgt diese Zurückführung druckerhöht. Hierzu kann gemäß den Ausführungsbeispielen der Fig. 1, 2 und 6 ein von der Anlage zur Synthese von Methanol 1 umfasster Recyclekompressor 25 zur Druckerhöhung des ersten Teilstroms 24 des weiteren Restgasstroms 23 vorgesehen sein.

Sinngemäß gleich wie für den Restgasstrom 18 können auch für den weiteren Restgasstrom 23 zusätzliche Teilströme vorgesehen sein. Gemäß der Darstellung der Fig. 1 bis 3 sowie 6 ist bevorzugt, dass ein zweiter Teilstrom 26 des weiteren Restgasstroms 23 gewonnen wird. Es kann dabei wie in den Fig. 1 bis 3 sowie 6 dargestellt sein, dass dieser zweite Teilstrom 26 prozesstechnisch einer Druckerhöhung des weiteren Restgasstroms 23 oder des ersten Teilstroms 24 des weiteren Restgasstroms 23 vorgelagert gewonnen wird. Mit anderen Worten hat dann der zweite Teilstrom 26 diese Druckerhöhung nicht erfahren, wodurch die entsprechende Vorrichtung zur Druckerhöhung wie speziell hier der Recyclekompressor 25 hinsichtlich seines Durchsatzes entlastet wird. Bevorzugt ist, dass der zweite Teilstrom 26 des weiteren Restgasstroms 23 der befeuerten Heizvorrichtung 17 zum Speisen der befeuerten Heizvorrichtung 17 zugeführt wird. Auf diese Weise kann ebenfalls verbliebenes Methan aus dem Synthesekreislauf der zweiten Methanolreaktoranordnung 8 entfernt werden.

In der Regel wird frisches Synthesegas mit einem Druck gewonnen, welcher für die Methanolsynthese nicht ausreicht oder jedenfalls nicht für einen wirtschaftlichen Betrieb der Methanolsynthese ausreicht. Daher ist bevorzugt vorgesehen, dass der erste Frischgasstrom 2 durch einen ersten Synthesegaskompressor 27 vor Zuführung zu der ersten Methanolreaktoranordnung 7 druckerhöht wird und dass der zweite Frischgasstrom 5 durch einen zweiten Synthesegaskompressor 28 vor Zuführung zu der zweiten Methanolreaktoranordnung 8 druckerhöht. Der erste Synthesegaskompressor 27 und der zweite Synthesegaskompressor 28 sind jeweils von der Anlage zur Synthese von Methanol 1 umfasst. Sowohl der erste Synthesegaskompressor 27 als auch der zweite Synthesegaskompressor 28 können jeweils eine Vielzahl von Kompressorstufen aufweisen. In einer bevorzugten Variante ist vorgesehen, dass der erste Teilstrom 19 des Restgasstroms 18 dem zweiten Frischgasstrom 5 zugeführt wird, wobei dies vorzugsweise prozesstechnisch dem zweiten Synthesegaskompressor 28 nachgelagert erfolgt. Dadurch wird der zweite Synthesegaskompressor 28 entlastet. In einer weiteren bevorzugten Variante weist der zweite Synthesegaskompressor 28 eine Vielzahl von Kompressorstufe auf und der erste Teilstrom 19 wird dem zweiten Frischgasstrom 5 prozesstechnisch zwischen zwei Kompressorstufen der Vielzahl von Kompressorstufen zugeführt.

Grundsätzlich kann der POX-Reaktor 6 auch mit Umgebungsluft betrieben werden. Eine bevorzugte Ausführung wie in den Fig. 1 bis 3 und 6 dargestellt sieht vor, dass dem POX-Reaktor 6 ein im Wesentlichen aus Sauerstoff bestehender O2-Strom 29 aus einer Luftzerlegungsvorrichtung 30 zum Gewinnen eines Stickstoffstroms 31 zugeführt wird. Der Stickstoffstrom 31 kann einer im Grunde beliebigen Verwendung zugeführt werden. Die Verwendung eines im Wesentlichen aus Sauerstoff bestehenden O2-Stroms 29 für den POX-Reaktor 6 führt u.a. dazu, dass der zweite Frischgasstrom 5 nur wenig Stickstoff aufweist, welcher Stickstoff für die Methanolsynthese inert ist. Das Vermeiden dieses Stickstoffs im zweiten Frischgasstrom 5 erlaubt es, die Dimensionierung der Kompressoren wie etwa des zweiten Synthesegaskompressors 28 zu verringern.

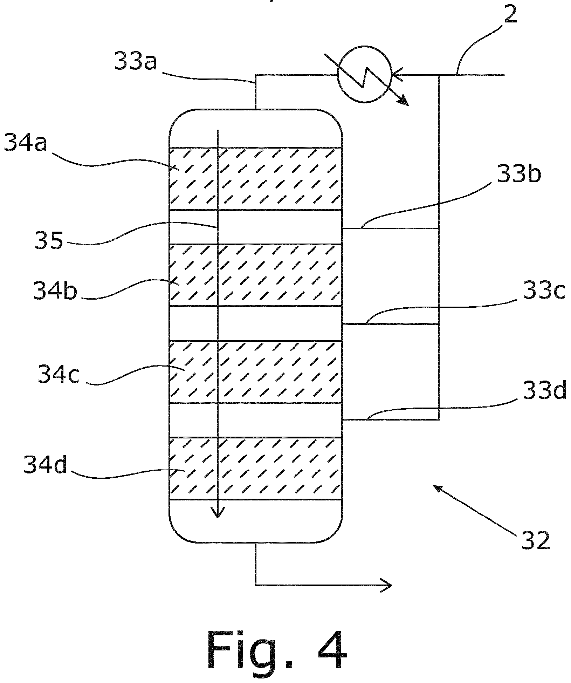

Grundsätzlich können sowohl die erste Methanolreaktoranordnung 7 als auch die zweite Methanolreaktoranordnung 8 beliebig ausgestaltet sein. Bevorzugt ist erstens, dass die erste Methanolreaktoranordnung 7 einen in der Fig. 4 genauer dargestellten adiabaten Quenchreaktor 32 zur Synthese von Methanol 1 aufweist. Der Quenchreaktor 32 weist eine Vielzahl von zueinander beabstandeten Einspeiseöffnungen 33a-d zur Zuführung des ersten Frischgasstroms 2 auf. Ebenso weist der Quenchreaktor eine Vielzahl von zueinander beabstandeten Katalysatorbetten 34a-d für die Synthese von Methanol 1 auf. Sowohl die Einspeiseöffnungen 33a-d als auch die Katalysatorbetten 34a-d sind vorzugsweise entlang einer Durchströmstrecke 35 des ersten Frischgasstroms 2 durch den Quenchreaktor 32 beabstandet. Durch diese Art der Anordnung kann der Temperaturanstieg durch die exotherme Methanolsynthesereaktion in dem Quenchreaktor 32 begrenzt werden. Ein solcher Quenchreaktor 32 eignet sich vorzugsweise dann, wenn die Stöchiometrie für die Methanolsynthese im Reaktor eher ungünstig ist, da ansonsten trotz der Parzellierung der Katalysatorbetten 34a-d der Temperaturanstieg nicht ausreichend begrenzt werden kann und in der Folge der Katalysator in Mitleidenschaft gezogen wird. Insofern bietet sich der Quenchreaktor 32 dann an, wenn im Wesentlichen das überstöchiometrische Synthesegas aus einer Dampfreformierung für die Methanolsynthese verwendet wird.

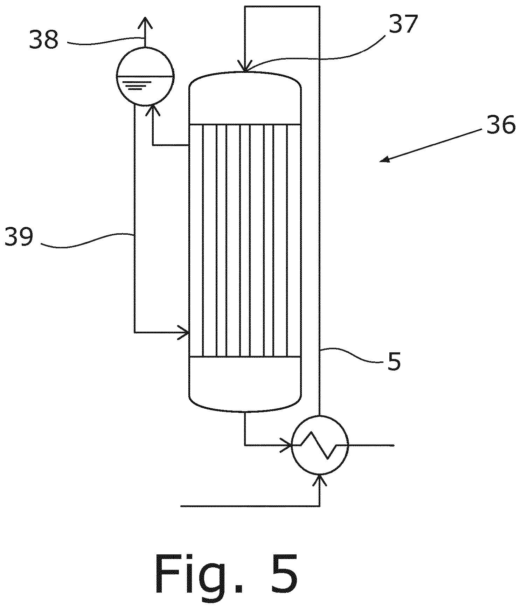

In diesem Zusammenhang ist es zweitens bevorzugt, dass die zweite Methanolreaktoranordnung 8 einen in der Fig. 5 abgebildeten Isothermreaktor 36 aufweist. Der Isothermreaktor 36 weist nur eine einzelne Zuführöffnung 37 zur Zuführung des zweiten Frischgasstroms 5 auf. Weiter wird im Isothermreaktor 36 die Reaktionswärme der Methanolsynthese bei Entstehung im Katalysatorbett des Isothermreaktor 36 an ein Wasserbad abgegeben. Dabei wird Mitteldruckdampf 38 gewonnen und abgeführt. Im Ergebnis entspricht die Temperatur im Katalysatorbett des Isothermreaktors 36 im Wesentlichen überall der Siedetemperatur bei dem Druck des Kühlwassers 39.

Grundsätzlich kann die vorschlagsgemäße Anlage zur Synthese von Methanol und das vorschlagsgemäße Verfahren zur Synthese von Methanol aus bloß einem Dampfreformer 3 und nur einem POX-Reaktor 6 mit der jeweils entsprechenden Methanolreaktoranordnung 7, 8 bestehen, was den Ausführungsbeispielen der Fig. 1, 2 und 6 entspricht. Bevorzugt ist jedoch, dass mehr als ein Dampfreformer 3 und entsprechend auch mehr als zwei Methanolreaktoranordnungen 7, 8 vorgesehen sind. Die Fig. 3 gibt ein solches Ausführungsbeispiel wieder.

Eine entsprechende bevorzugte Ausführungsform ist dadurch gekennzeichnet, dass mindestens ein weiterer Frischgasstrom 40 mit Wasserstoff und Kohlenstoffoxiden in mindestens einem weiteren Dampfreformer 41 durch Dampfreformierung aus dem kohlenstoffhaltigen Energieträgerstrom 4 gewonnen wird, dass der mindestens eine weitere Frischgasstrom 40 mindestens einer weiteren Methanolreaktoranordnung 42 zur Synthese von Methanol 1 zugeführt wird, dass jeweils ein weiterer Restgasstrom 43 mit unreagiertem Wasserstoff und unreagierten Kohlenstoffoxiden aus der mindestens einen weiteren Methanolreaktoranordnung 42 gewonnen wird und dass zumindest ein Teil des unreagierten Wasserstoffs und der unreagierten Kohlenstoffoxide des jeweiligen weiteren Restgasstroms 43 der zweiten Methanolreaktoranordnung 8 zur Synthese von Methanol 1 zugeführt wird. Das Ausführungsbeispiel der Fig. 3 zeigt eine Anlage zur Synthese von Methanol 1, welche genau einen weiteren Dampfreformer 41 und genau eine weitere Methanolreaktoranordnung 42 zur Synthese von Methanol 1 aufweist, sodass jeweils genau ein weiterer Frischgasstrom 40 und genau ein weiterer Restgasstrom 43 gewonnen wird.

Bevorzugt ist weiter, dass die mindestens eine weitere Methanolreaktoranordnung 43 jeweils eine weitere Kondensationsvorrichtung 44 zum Erhalten eines jeweiligen weiteren Rohmethanolstroms 45 aufweist, welcher einer jeweiligen weiteren Destillationsanordnung 46 zum Gewinnen eines jeweiligen weiteren Methanolproduktstroms 47 umfassend Methanol 1 zugeführt wird.

Ein erster Teilstrom 50 des weiteren Restgasstroms 43 wird bevorzugt und wie in der Fig. 3 dargestellt der zweiten Methanolreaktoranordnung 8 zur Synthese von Methanol 1 zugeführt. Ein zweiter Teilstrom 49 des weiteren Restgasstroms 43 wird hingegen zur weiteren Methanolreaktoranordnung 42 zurückgeführt.

Die Anlage zur Synthese von Methanol 1 der Fig. 3 weist daneben bevorzugt und wie dargestellt für die zweite Methanolreaktoranordnung 8 und die weitere Methanolreaktoranordnung 42 einen jeweils einen Zirkulationskompressor 21 bzw. einen weiteren Zirkulationskompressor 48 auf. Dabei ist der weitere Zirkulationskompressor 48 für eine Druckerhöhung des zweiten Teilstroms 49 des weiteren Restgasstroms 43 eingerichtet.

Ferner weist die Anlage zur Synthese von Methanol 1 der Fig. 3 wie dargestellt und ebenfalls bevorzugt einen Sammelkompressor 51 auf, welcher sowohl den ersten Teilstrom 19 des Restgasstroms 18 als auch den ersten Teilstrom 50 des weiteren Restgasstroms 43 nach ihrer Zusammenführung druckerhöht. Nach Zusammenführung und Druckerhöhung werden der erste Teilstrom 19 des Restgasstroms 18 und der erste Teilstrom 50 des weiteren Restgasstroms 43 vorzugsweise mit dem zweiten Frischgasstrom 5 zusammengeführt.

Das vorschlagsgemäße Verfahren zum Umbau einer Anlage zur Synthese von Methanol 1, welche Anlage mindestens einen Dampfreformer 3, 41 zum Gewinnen eines jeweiligen Frischgasstroms 2, 5, 40 mit Wasserstoff und Kohlenstoffoxiden durch Dampfreformierung aus einem kohlenstoffhaltigen Energieträgerstrom 4 und mindestens eine Methanolreaktoranordnung 7, 8 zur Synthese von Methanol 1 aus einem jeweiligen Frischgasstrom 2, 5, 40 aufweist, ist dadurch gekennzeichnet, dass ein POX-Reaktor 6 zum Gewinnen eines Frischgasstroms 2, 5, 40 der Anlage hinzugefügt wird.

Wie soeben festgestellt kann es sein, dass die Anlage genau einen Dampfreformer 3, 41 und genau eine Methanolreaktoranordnung 7, 8 aufweist. Bevorzugt ist jedoch, dass die Anlage eine Vielzahl von Dampfreformern 3, 41 zum Gewinnen eines jeweiligen Frischgasstroms 2, 5, 40 mit Wasserstoff und Kohlenstoffoxiden durch Dampfreformierung aus einem kohlenstoffhaltigen Energieträgerstroms 4 und eine Vielzahl von Methanolreaktoranordnungen 7, 8 zur Synthese von Methanol 1 aus einem jeweiligen Frischgasstrom 2, 5, 40 aufweist.

Dieses Hinzufügen des POX-Reaktors 6 kann dabei auf unterschiedliche Art und Weise erfolgen. In einer ersten bevorzugten Variante, bei welcher die Anlage eine Vielzahl von Dampfreformern 3, 41 und eine Vielzahl von Methanolreaktoranordnungen 7, 8 aufweist, wird ein Dampfreformer 3, 41 der Vielzahl von Dampfreformern 3, 41 durch einen POX-Reaktor 6 zum Gewinnen des jeweiligen Frischgasstroms 2, 5, 40 ersetzt.

Grundsätzlich kann die Methanolreaktoranordnung 7, 8 in dem Strang des ersetzten Dampfreformers 3, 41 dabei beibehalten werden. Bevorzugt ist jedoch, dass daneben auch eine Methanolreaktoranordnung 7, 8 der Vielzahl von Methanolreaktoranordnungen 7, 8 durch eine andere Methanolreaktoranordnung 7, 8 ersetzt wird. Diese andere Methanolreaktoranordnung 7, 8 kann insbesondere einen Isothermreaktor 36, wie er etwa in der Fig. 5 dargestellt wird, umfassen.

Vorteilhafterweise werden Kompressoren der Anlage bei dem Hinzufügen des POX-Reaktors 6 beibehalten. Insbesondere ist bevorzugt, dass der Zirkulationskompressor 21, der Recyclekompressor 25, der erste Synthesegaskompressor 27, der zweite Synthesegaskompressor 28 und/oder der weitere Zirkulationskompressor 48 beibehalten werden.

Statt dem Ersatz eines Dampfreformers 3, 41 innerhalb eines bestehenden Strangs kann das Hinzufügen des POX-Reaktor 6 auch das Hinzufügen eines neuen Strangs bedeuten. Eine bevorzugte Variante sieht daher vor, dass auch eine weitere Methanolreaktoranordnung 7, 8 zur Synthese von Methanol 1 der Anlage hinzugefügt wird und dass der aus dem POX-Reaktor 6 gewonnene Fischgasstrom 2, 5, 40 der hinzugefügten weiteren Methanolreaktoranordnung 7, 8 zugeführt wird.

Gemäß einer bevorzugten Variante des Verfahrens zum Umbau einer Anlage zur Synthese von Methanol 1 werden der mindestens eine Dampfreformer 3, 41 zum Gewinnen eines jeweiligen Frischgasstroms 2, 5, 40 mit Wasserstoff und Kohlenstoffoxiden und die mindestens eine Methanolreaktoranordnung 7, 8 zur Synthese von Methanol 1 während des Hinzufügens des POX-Reaktors 6 im Wesentlichen weiterbetrieben. Dies bedeutet, dass der Betrieb des mindestens einen Dampfreformers 3, 41 und der mindestens einen Methanolreaktoranordnung 7, 8 erst in dem Augenblick angepasst oder unterbrochen werden muss, in dem der POX-Reaktor 6 selbst den Betrieb aufnimmt und aktiver Bestandteil der Anlage wird. Diese Möglichkeit betrifft insbesondere die Variante, bei der mit dem POX-Reaktor 6 auch die weitere Methanolreaktoranordnung 7, 8 zur Synthese von Methanol 1 der Anlage hinzugefügt wird und dass der aus dem POX-Reaktor 6 gewonnene Fischgasstrom 2, 5, 40 der hinzugefügten weiteren Methanolreaktoranordnung 7, 8 zugeführt wird.

Die Anlagen zur Synthese von Methanol 1 der Fig. 1 bis 3 und 6 können jeweils als Ergebnis des vorschlagsgemäßen Verfahrens zum Umbau einer Anlage zur Synthese von Methanol 1 verstanden. Speziell der POX-Reaktor 6 und die zweite Methanolreaktoranordnung 8, welche einen Isothermreaktor 36 aufweist, ersetzen einen zuvor vorgesehenen Dampfreformer und eine zuvor vorgesehene Methanolreaktoranordnung mit einem Quenchreaktor.

Die vorschlagsgemäße Anlage zur Synthese von Methanol 1 weist einen Dampfreformer 3 zum Gewinnen eines ersten Frischgasstroms 2 mit Wasserstoff und Kohlenstoffoxiden durch Dampfreformierung aus einem kohlenstoffhaltigen Energieträgerstrom 4 auf. Die vorschlagsgemäße Anlage zur Synthese von Methanol 1 weist ebenso einen POX-Reaktor 6 zum Gewinnen eines zweiten Frischgasstroms 5 mit Wasserstoff und Kohlenstoffoxiden durch partielle Oxidation aus dem kohlenstoffhaltigen Energieträgerstrom 4. Die vorschlagsgemäße Anlage zur Synthese von Methanol 1 weist ferner eine erste Methanolreaktoranordnung 7 zur Synthese von Methanol 1 aus dem ersten Frischgasstrom 2.

Die vorschlagsgemäße Anlage zur Synthese von Methanol 1 ist dadurch gekennzeichnet, dass die Anlage eine zweite Methanolreaktoranordnung 8 zur Synthese von Methanol 1 aus dem zweiten Frischgasstrom 5 aufweist.

Die vorschlagsgemäße Anlage zur Synthese von Methanol 1 ist weiter dadurch gekennzeichnet, dass ein Restgasstrom 18 mit unreagiertem Wasserstoff und unreagierten Kohlenstoffoxiden aus der ersten Methanolreaktoranordnung 7 gewonnen wird und dass zumindest ein Teil des unreagierten Wasserstoffs des Restgasstroms 18 der zweiten Methanolreaktoranordnung 8 zur Synthese von Methanol 1 zugeführt wird.

## Patentansprüche

1. Verfahren zur Synthese von Methanol (1), wobei ein erster Frischgasstrom (2) mit Wasserstoff und Kohlenstoffoxiden in einem Dampfreformer (3) durch Dampfreformierung aus einem kohlenstoffhaltigen Energieträgerstrom (4) gewonnen wird, wobei ein zweiter Frischgasstrom (5) mit Wasserstoff und Kohlenstoffoxiden in einem POX-Reaktor (6) durch partielle Oxidation aus dem kohlenstoffhaltigen Energieträgerstrom (4) gewonnen wird, wobei der erste Frischgasstrom (2) einer ersten Methanolreaktoranordnung (7) zur Synthese von Methanol (1) zugeführt wird, **dadurch gekennzeichnet, dass** der zweite Frischgasstrom (5) einer zweiten Methanolreaktoranordnung (8) zur Synthese von Methanol (1) zugeführt wird, dass ein Restgasstrom (18) mit unreagiertem Wasserstoff und unreagierten Kohlenstoffoxiden aus der ersten Methanolreaktoranordnung (7) gewonnen wird und dass zumindest ein Teil des unreagierten Wasserstoffs des Restgasstroms (18) der zweiten Methanolreaktoranordnung (8) zur Synthese von Methanol (1) zugeführt wird.

2. Verfahren nach Anspruch 1, **dadurch gekennzeichnet, dass** der Dampfreformer (3) durch eine befeuerte Heizvorrichtung (17) erhitzt wird, vorzugsweise, dass die befeuerte Heizvorrichtung (17) durch den kohlenstoffhaltigen Energieträgerstrom (4) gespeist wird.

3. Verfahren nach Anspruch 1 oder 2, **dadurch gekennzeichnet, dass** auch zumindest ein Teil der unreagierten Kohlenstoffoxide des Restgasstroms (18) der zweiten Methanolreaktoranordnung (8) zur Synthese von Methanol (1) zugeführt wird.

4. Verfahren nach einem der Ansprüche 1 bis 3, **dadurch gekennzeichnet, dass** ein erster Teilstrom (19) des Restgasstroms (18) insbesondere druckerhöht der zweiten Methanolreaktoranordnung (8) zur Synthese von Methanol (1) zugeführt wird, vorzugsweise, dass im Wesentlichen der vollständige erste Teilstrom (19), insbesondere mit im Wesentlichen gleichbleibender Zusammensetzung, der zweiten Methanolreaktoranordnung (8) zur Synthese von Methanol (1) zugeführt wird.

5. Verfahren nach einem der Ansprüche 1 bis 3, **dadurch gekennzeichnet, dass** ein erster Teilstrom (19) des Restgasstroms (18) dem POX-Reaktor (6) zum Gewinnen des zweiten Frischgasstroms (5) zugeführt wird.

6. Verfahren nach einem der Ansprüche 1 bis 3, **dadurch gekennzeichnet, dass** ein erster Teilstrom (19) des Restgasstroms (18) einer Wasserstoffrückgewinnungsanordnung (52) zum Gewinnen eines wasserstoffhaltigen Stroms (53) und eines Reststroms (54) zugeführt wird, vorzugsweise, dass der wasserstoffhaltige Strom (53) der zweiten Methanolreaktoranordnung (8) zur Synthese von Methanol (1) zugeführt wird, insbesondere, dass der wasserstoffhaltige Strom (53) im Wesentlichen aus Wasserstoff besteht.

7. Verfahren nach einem der Ansprüche 1 bis 6, dass ein zweiter Teilstrom (20) des Restgasstroms (18) zur ersten Methanolreaktoranordnung (7) zur Synthese von Methanol (1) weiter vorzugsweise druckerhöht zurückgeführt wird.

8. Verfahren nach einem der Ansprüche 1 bis 7, **dadurch gekennzeichnet, dass** ein dritter Teilstrom (22) des Restgasstroms (18) aus dem Restgasstrom (18) gewonnen wird, weiter vorzugsweise, dass der dritte Teilstrom (22) des Restgasstroms (18) der befeuerten Heizvorrichtung (17) zum Speisen der befeuerten Heizvorrichtung (17) zugeführt wird.

9. Verfahren nach Anspruch 8, **dadurch gekennzeichnet, dass** ein Massendurchsatz des dritten Teilstroms (22) niedriger ist als ein Massendurchsatz des ersten Teilstroms (19), vorzugsweise, dass der Massendurchsatz des dritten Teilstroms (22) zwischen 25 % und 70 % des Massendurchsatzes des ersten Teilstroms (19) beträgt, insbesondere, dass der Massendurchsatz des dritten Teilstroms (22) zwischen 40 % und 50 % des Massendurchsatzes des ersten Teilstroms (19) beträgt.

10. Verfahren nach einem der Ansprüche 1 bis 9, **dadurch gekennzeichnet, dass** ein weiterer Restgasstrom (23) mit unreagiertem Wasserstoff und unreagierten Kohlenstoffoxiden aus der zweiten Methanolreaktoranordnung (8) gewonnen wird und dass ein erster Teilstrom (24) des weiteren Restgasstroms (23) insbesondere druckerhöht zur zweiten Methanolreaktoranordnung (8) zur Synthese von Methanol (1) zurückgeführt wird.

11. Verfahren nach Anspruch 10, **dadurch gekennzeichnet, dass** ein zweiter Teilstrom (26) des weiteren Restgasstroms (23), vorzugsweise prozesstechnisch einer Druckerhöhung des weiteren Restgasstroms (23) vorgelagert, gewonnen wird, vorzugsweise, dass der zweite Teilstrom (26) des weiteren Restgasstroms (23) der befeuerten Heizvorrichtung (17) zum Speisen der befeuerten Heizvorrichtung (17) zugeführt wird.

12. Verfahren nach einem der Ansprüche 1 bis 11, **dadurch gekennzeichnet, dass** der erste Frischgasstrom (2) durch einen ersten Synthesegaskompressor (27) vor Zuführung zu der ersten Methanolreaktoranordnung (7) druckerhöht wird und dass der zweite Frischgasstrom (5) durch einen zweiten Synthesegaskompressor (28) vor Zuführung zu der zweiten Methanolreaktoranordnung (8) druckerhöht wird, vorzugsweise, dass der erste Teilstrom (19) des Restgasstroms (18) dem zweiten Frischgasstrom (5) zugeführt wird.

13. Verfahren nach einem der Ansprüche 1 bis 12, **dadurch gekennzeichnet, dass** dem POX-Reaktor (6) ein im Wesentlichen aus Sauerstoff bestehender O2-Strom (29) aus einer Luftzerlegungsvorrichtung (30) zum Gewinnen eines Stickstoffstroms (31) zugeführt wird.

14. Verfahren nach einem der Ansprüche 1 bis 13, **dadurch gekennzeichnet, dass** mindestens ein weiterer Frischgasstrom (40) mit Wasserstoff und Kohlenstoffoxiden in mindestens einem weiteren Dampfreformer (41) durch Dampfreformierung aus dem kohlenstoffhaltigen Energieträgerstrom (4) gewonnen wird, dass der mindestens eine weitere Frischgasstrom (40) mindestens einer weiteren Methanolreaktoranordnung (42) zur Synthese von Methanol (1) zugeführt wird und dass jeweils ein weiterer Restgasstrom (43) mit unreagiertem Wasserstoff und unreagierten Kohlenstoffoxiden aus der mindestens einen weiteren Methanolreaktoranordnung (42) gewonnen wird und dass zumindest ein Teil des unreagierten Wasserstoffs und der unreagierten Kohlenstoffoxide des jeweiligen weiteren Restgasstroms (43) der zweiten Methanolreaktoranordnung (8) zur Synthese von Methanol (1) zugeführt wird.

15. Verfahren zum Umbau einer Anlage zur Synthese von Methanol (1), welche Anlage mindestens einen Dampfreformer (3, 41) zum Gewinnen eines jeweiligen Frischgasstroms (2, 5, 40) mit Wasserstoff und Kohlenstoffoxiden durch Dampfreformierung aus einem kohlenstoffhaltigen Energieträgerstrom (4) und mindestens eine Methanolreaktoranordnung (7, 8) zur Synthese von Methanol (1) aus einem jeweiligen Frischgasstrom (2, 5, 40) aufweist, **dadurch gekennzeichnet, dass** ein POX-Reaktor (6) zum Gewinnen eines Frischgasstroms (2, 5, 40) der Anlage hinzugefügt wird.

16. Anlage zur Synthese von Methanol (1) mit einem Dampfreformer (3) zum Gewinnen eines ersten Frischgasstroms (2) mit Wasserstoff und Kohlenstoffoxiden durch Dampfreformierung aus einem kohlenstoffhaltigen Energieträgerstrom (4) mit einem POX-Reaktor (6) zum Gewinnen eines zweiten Frischgasstroms (5) mit Wasserstoff und Kohlenstoffoxiden durch partielle Oxidation aus dem kohlenstoffhaltigen Energieträgerstrom (4) und einer ersten Methanolreaktoranordnung (7) zur Synthese von Methanol (1) aus dem ersten Frischgasstrom (2), **dadurch gekennzeichnet, dass** die Anlage eine zweite Methanolreaktoranordnung (8) zur Synthese von Methanol (1) aus dem zweiten Frischgasstrom (5) aufweist, dass ein Restgasstrom (18) mit unreagiertem Wasserstoff und unreagierten Kohlenstoffoxiden aus der ersten Methanolreaktoranordnung (7) gewonnen wird und dass zumindest ein Teil des unreagierten Wasserstoffs des Restgasstroms (18) der zweiten Methanolreaktoranordnung (8) zur Synthese von Methanol (1) zugeführt wird.

## Claims

1. A method for the synthesis of methanol (1), wherein a first fresh gas flow (2) comprising hydrogen and carbon oxides is obtained from a carbonaceous energy carrier flow (4) in a steam reformer (3) by steam reforming, wherein a second fresh gas flow (5) comprising hydrogen and carbon oxides is obtained from the carbonaceous energy carrier flow (4) in a POX reactor (6) by partial oxidation, wherein the first fresh gas flow (2) is supplied to a first methanol reactor stage (7) for the synthesis of methanol (1), **characterized in that** the second fresh gas flow (5) is supplied to a second methanol reactor stage (8) for the synthesis of methanol (1), **in that** a residual gas flow (18) comprising unreacted hydrogen and unreacted carbon oxides is obtained from the first methanol reactor stage (7) and **in that** at least part of the unreacted hydrogen of the residual gas flow (18) is supplied to the second methanol reactor stage (8) for the synthesis of methanol (1).

2. The method according to Claim 1, **characterized in that** the steam reformer (3) is heated by a fired heating device (17), preferably that the fired heating device (17) is fed by the carbonaceous energy carrier flow (4) .

3. The method according to Claim 1 or 2, **characterized in that** at least a portion of the unreacted carbon oxides of the residual gas flow (18) is supplied to the second methanol reactor stage (8) for the synthesis of methanol (1).

4. The method according to one of Claims 1 to 3, **characterized in that** a first partial flow (19) of the residual gas flow (18) is supplied, particularly in a pressure-boosted manner, to the second methanol reactor stage (8) for the synthesis of methanol (1), preferably **in that** substantially the entire first partial flow (19), in particular with a substantially uniform composition, is supplied to the second methanol reactor stage (8) for the synthesis of methanol (1).

5. The method according to one of Claims 1 to 3, **characterized in that** a first partial flow (19) of the residual gas flow (18) is supplied to the POX reactor (6), in order to obtain the second fresh gas flow (5).

6. The method according to one of Claims 1 to 3, **characterized in that** a first partial flow (19) of the residual gas flow (18) is supplied to a hydrogen recovery stage (52), in order to obtain a hydrogen-containing flow (53) and a residual flow (54), preferably that the hydrogen-containing flow (53) is supplied to the second methanol reactor stage (8) for the synthesis of methanol (1), in particular that the hydrogen-containing flow (53) substantially consists of hydrogen.

7. The method according to one of Claims 1 to 6, that a second partial flow (20) of the residual gas flow (18) is fed back to the first methanol reactor stage (7) for the synthesis of methanol (1), more preferably in a pressure-boosted manner.

8. The method according to one of Claims 1 to 7, **characterized in that** a third partial flow (22) of the residual gas flow (18) is obtained from the residual gas flow (18), more preferably that the third partial flow (22) of the residual gas flow (18) is supplied to the fired heating device (17), in order to feed said fired heating device (17).

9. The method according to Claim 8, **characterized in that** a mass throughput of the third partial flow (22) is lower than a mass throughput of the first partial flow (19), preferably that the mass throughput of the third partial flow (22) is between 25% and 70% of the mass throughput of the first partial flow (19), in particular that the mass throughput of the third partial flow (22) is between 40% and 50% of the mass throughput of the first partial flow (19).

10. The method according to one of Claims 1 to 9, **characterized in that** a further residual gas flow (23) comprising unreacted hydrogen and unreacted carbon oxides is obtained from the second methanol reactor stage (8) and that a first partial flow (24) of the further residual gas flow (23) is fed back to the second methanol reactor stage (8) for the synthesis of methanol (1), in particular in a pressure-boosted manner.

11. The method according to Claim 10, **characterized in that** a second partial flow (26) of the further residual gas flow (23) is obtained, preferably upstream in terms of process technology of a pressure increase in the further residual gas flow (23), preferably that the second partial flow (26) of the further residual gas flow (23) is supplied to the fired heating device (17), in order to feed said fired heating device (17).

12. The method according to one of Claims 1 to 11, **characterized in that** the first fresh gas flow (2) is pressure-boosted by a first synthesis gas compressor (27) before being supplied to the first methanol reactor stage (7) and that the second fresh gas flow (5) is pressure-boosted by a second synthesis gas compressor (28) before being supplied to the second methanol reactor stage (8), preferably that the first partial flow (19) of the residual gas flow (18) is supplied to the second fresh gas flow (5).

13. The method according to one of Claims 1 to 12, **characterized in that** an O2 flow (29) substantially consisting of oxygen is supplied to the POX reactor (6) from an air separation device (30), in order to obtain a nitrogen flow (31).

14. Method according to one of Claims 1 to 13, **characterized in that** at least one further fresh gas flow (40) comprising hydrogen and carbon oxides is obtained from the carbonaceous energy carrier flow (4) in at least one further steam reformer (41) through steam reformation, that the at least one further fresh gas flow (40) is supplied to at least one further methanol reactor stage (42) for the synthesis of methanol (1) and that a further residual gas flow (43) comprising unreacted hydrogen and unreacted carbon oxides is obtained from the at least one further methanol reactor stage (42) and that at least part of the unreacted hydrogen and the unreacted carbon oxides of the further residual gas flow (43) in each case is supplied to the second methanol reactor stage (8) for the synthesis of methanol (1).

15. A method for converting a plant for the synthesis of methanol (1), which plant comprises at least one steam reformer (3, 41) for obtaining a respective fresh gas flow (2, 5, 40) comprising hydrogen and carbon oxides by steam reforming from a carbonaceous energy carrier flow (4) and at least one methanol reactor stage (7, 8) for the synthesis of methanol (1) from a fresh gas flow (2, 5, 40) in each case, **characterized in that** a POX reactor (6) for obtaining a fresh gas flow (2, 5, 40) is added to the plant.

16. A plant for the synthesis of methanol (1) comprising a steam reformer (3) for obtaining a first fresh gas flow (2) comprising hydrogen and carbon oxides from a carbonaceous energy carrier flow (4) by steam reforming with a POX reactor (6) for obtaining a second fresh gas flow (5) comprising hydrogen and carbon oxides through partial oxidation from the carbonaceous energy carrier flow (4) and a first methanol reactor stage (7) for the synthesis of methanol (1) from the first fresh gas flow (2), **characterized in that** the plant comprises a second methanol reactor stage (8) for the synthesis of methanol (1) from the second fresh gas flow (5), that a residual gas flow (18) with unreacted hydrogen and unreacted carbon oxides is obtained from the first methanol reactor stage (7) and that at least part of the unreacted hydrogen of the residual gas flow (18) is supplied to the second methanol reactor stage (8) for the synthesis of methanol (1).

## Revendications

1. Procédé de synthèse de méthanol (1), dans lequel un premier flux de gaz frais (2) comportant de l'hydrogène et des oxydes de carbone est obtenu dans un dispositif de reformage à la vapeur (3) en soumettant un flux vecteur d'énergie (4) contenant du carbone à un reformage à la vapeur, un deuxième flux de gaz frais (5) comportant de l'hydrogène et des oxydes de carbone étant obtenu dans un réacteur POX (6) en soumettant le flux vecteur d'énergie (4) à une oxydation partielle, le premier flux de gaz frais (2) étant introduit dans un premier système de réacteur de méthanol (7) afin de synthétiser du méthanol (1), **caractérisé en ce que** le deuxième flux de gaz frais (5) est introduit dans un deuxième système de réacteur de méthanol (8) afin de synthétiser du méthanol (1), qu'un flux de gaz résiduel (18) comportant de l'hydrogène n'ayant pas réagi et des oxydes de carbone n'ayant pas réagi est obtenu à partir du premier système de réacteur de méthanol (7) et qu'au moins une partie de l'hydrogène n'ayant pas réagi contenu dans le flux de gaz résiduel (18) est introduite dans le deuxième système de réacteur de méthanol (8) afin de synthétiser du méthanol (1).

2. Procédé selon la revendication 1, **caractérisé en ce que** le dispositif de reformage à la vapeur (3) est chauffé par un dispositif de chauffage (17) à flamme, le dispositif de chauffage (17) à flamme étant préférentiellement alimenté par le flux vecteur d'énergie (4) contenant du carbone.

3. Procédé selon les revendications 1 ou 2, **caractérisé en ce qu'**au moins une partie des oxydes de carbone n'ayant pas réagi contenus dans le flux de gaz résiduel (18) étant également introduite dans le système de réacteur de méthanol (8) afin de synthétiser du méthanol (1).

4. Procédé selon l'une des revendications 1 à 3, **caractérisé en ce qu'**un premier flux partiel (19) du flux de gaz résiduel (18) est introduit, notamment en augmentant sa pression, dans le deuxième système de réacteur de méthanol (8) afin de synthétiser du méthanol (1), que le premier flux partiel (19) est de préférence introduit, sensiblement dans son intégralité et notamment avec une composition restant sensiblement constante, dans le système de réacteur de méthanol (8) afin de synthétiser du méthanol (1).

5. Procédé selon l'une des revendications 1 à 3, **caractérisé en ce qu'**un premier flux partiel (19) du flux de gaz résiduel (18) est introduit dans le réacteur POX (6) afin d'obtenir le deuxième flux de gaz frais (5) .

6. Procédé selon l'une des revendications 1 à 3, **caractérisé en ce qu'**un premier flux partiel (19) du flux de gaz résiduel (18) est introduit dans un système de récupération d'hydrogène (52) afin d'obtenir un flux (53) contenant de l'hydrogène et un flux résiduel (54), que le flux (53) contenant de l'hydrogène est préférentiellement introduit dans le deuxième système de réacteur de méthanol (8) afin de synthétiser du méthanol (1), que le flux (53) contenant de l'hydrogène est notamment constitué sensiblement d'hydrogène.

7. Procédé selon l'une des revendications 1 à 6, **caractérisé en ce qu'**un deuxième flux partiel (20) du flux de gaz résiduel (18) est recyclé, de préférence encore en augmentant sa pression, dans le premier système de réacteur de méthanol (7) afin de synthétiser du méthanol (1).

8. Procédé selon l'une des revendications 1 à 7, **caractérisé en ce qu'**un troisième flux partiel (22) du flux de gaz résiduel (18) est obtenu à partir du flux de gaz résiduel (18), que le troisième flux partiel (22) du flux de gaz résiduel (18) est introduit, de préférence encore, dans le dispositif de chauffage (17) à flamme afin d'alimenter le dispositif de chauffage (17) à flamme.

9. Procédé selon la revendication 8, **caractérisé en ce qu'**un débit massique du troisième flux partiel (22) est inférieur à un débit massique du premier flux partiel (19), que le débit massique du troisième flux partiel (22) est préférentiellement compris entre 25 % et 70 % du débit massique du premier flux partiel (19), que le débit massique du troisième flux partiel (22) est notamment compris entre 40 % et 50 % du débit massique du premier flux partiel (19).

10. Procédé selon l'une des revendications 1 à 9, **caractérisé en ce que** l'on obtient, à partir du deuxième système de réacteur de méthanol (8), un flux de gaz résiduel (23) supplémentaire comportant de l'hydrogène n'ayant pas réagi et des oxydes de carbone n'ayant pas réagi et qu'un premier flux partiel (24) du flux de gaz résiduel (23) supplémentaire est introduit, notamment en augmentant sa pression, dans le deuxième système de réacteur de méthanol (8) afin de synthétiser du méthanol (1) .

11. Procédé selon la revendication 10, **caractérisé en ce que** l'on obtient un deuxième flux partiel (26) du flux de gaz résiduel (23) supplémentaire , de préférence en amont de l'endroit où le flux de gaz résiduel supplémentaire (23) subit une augmentation de sa pression, que le deuxième flux partiel (26) du flux de gaz résiduel (23) supplémentaire est introduit préférentiellement dans le dispositif de chauffage (17) à flamme afin d'alimenter le dispositif de chauffage (17) à flamme.

12. Procédé selon l'une des revendications 1 bis 11, **caractérisé en ce que** le premier flux de gaz frais (2) subit, en amont de son introduction dans le premier système de réacteur de méthanol (7), une augmentation de sa pression au moyen d'un premier compresseur de gaz de synthèse (27), et que le deuxième flux de gaz frais (5) subit, en amont de son introduction dans le deuxième système de réacteur de méthanol (8), une augmentation de sa pression au moyen d'un deuxième compresseur de gaz de synthèse (28), que le premier flux partiel (19) du flux de gaz résiduel (18) est de préférence introduit dans le deuxième flux de gaz frais (5) .

13. Procédé selon l'une des revendications 1 à 12, **caractérisé en ce que** l'on introduit un flux d'O2 (29), lequel est sensiblement constitué d'oxygène et lequel est issu d'un dispositif de liquéfaction fractionnée d'air (30) destiné à obtenir un flux d'azote (31), dans le réacteur POX (6).

14. Procédé selon l'une des revendications 1 à 13, **caractérisé en ce qu'**au moins un flux de gaz frais (40) supplémentaire comportant de l'hydrogène et des oxydes de carbone est obtenu en soumettant, dans au moins un dispositif de reformage à la vapeur (41) supplémentaire, le flux vecteur d'énergie (4) contenant du carbone à un reformage à la vapeur, que l'au moins un flux de gaz frais (40) supplémentaire est introduit dans au moins un système de réacteur de méthanol (42) supplémentaire afin de synthétiser du méthanol (1), et que l'on obtient, à chaque instance, un flux de gaz résiduel (43) supplémentaire lequel comporte de l'hydrogène n'ayant pas réagi et des oxydes de carbone n'ayant pas réagi et lequel est issu de l'au moins un système de réacteur de méthanol (42) supplémentaire, et qu'au moins une partie de l'hydrogène n'ayant pas réagi et des oxydes de carbone n'ayant pas réagi, lesquels sont issus du flux de gaz résiduel (43) supplémentaire concerné, est introduite dans le deuxième système de réacteur de méthanol (8) afin de synthétiser du méthanol (1) .

15. Procédé de modification d'une installation de synthèse de méthanol (1), ladite installation comportant au moins un dispositif de reformage à la vapeur (3, 41) dont chacun est destiné à obtenir, en soumettant un flux vecteur d'énergie (4) contenant du carbone à un reformage à la vapeur, un flux de gaz frais (2, 5, 40) comportant de l'hydrogène et des oxydes de carbone, et au moins un système de réacteur de méthanol (7, 8) destiné à synthétiser du méthanol (1) à partir du flux de gaz frais (2, 5, 40) concerné, **caractérisé en ce que** l'on ajoute à ladite installation un réacteur POX (6) destiné à obtenir un flux de gaz frais (2, 5, 40).

16. Installation de synthèse de méthanol (1) pourvue d'un dispositif de reformage à la vapeur (3) destiné à obtenir, en soumettant un flux vecteur d'énergie (4) contenant du carbone à un reformage à la vapeur, un premier flux de gaz frais (2) comportant de l'hydrogène et des oxydes de carbone d'un réacteur POX (6) destiné à obtenir, en soumettant le flux vecteur d'énergie (4) contenant du carbone à une oxydation partielle, un deuxième flux de gaz frais (5) comportant de l'hydrogène et des oxydes de carbone, et d'un premier système de réacteur de méthanol (7) destiné à synthétiser du méthanol (1) à partir du premier flux de gaz frais (2), **caractérisée en ce que** ladite installation comporte un deuxième système de réacteur de méthanol (8) destiné à synthétiser du méthanol (1) à partir du deuxième flux de gaz frais (5), que l'on obtient, à partir du premier système de réacteur de méthanol (7), un flux de gaz résiduel (18) comportant de l'hydrogène n'ayant pas réagi et des oxydes de carbone n'ayant pas réagi, et que l'on introduit au moins une partie de l'hydrogène, qui n'a pas réagi et qui est issu du flux de gaz résiduel (18), dans le deuxième système de réacteur de méthanol (8) afin de synthétiser du méthanol (1).
